# EUROPÄISCHE PATENTANMELDUNG

(11) **EP 1 096 257 A2**
(43) Veröffentlichungstag der Anmeldung: **02.05.2001**
(21) Anmeldenummer: 00123053.1
(22) Anmeldetag: 24.10.2000
(51) Int. Cl.: G01N 33/58, G01N 33/537, G01N 33/68, G01N 33/543, C07K 1/13, G01N 33/532

(54) **Verfahren, Vorrichtung und farbstoffmarkiertes Peptid zum Nachweis eines Moleküls**

(30) Priorität: 29.10.1999 DE 19952160
(71) Anmelder: Sauer, Markus, 68124 Heidelberg (DE); Wolfrum, Jürgen, Prof. Dr., 37124 Rosdorf-Obernjesa (DE)
(72) Erfinder: Sauer, Markus, 68124 Heidelberg (DE); Wolfrum, Jürgen, Prof. Dr., 37124 Rosdorf-Obernjesa (DE)
(74) Vertreter: Köllner, Malte, Dr.

(57) **Zusammenfassung**

Das Verfahren ist bei einem Ausführungsbeispiel in der Lage, einen Tumormarker (32) des Mammakarzinoms zu detektieren, nämlich das für das Mammakarzinom spezifische, unterglykosilierte MUC1-Glykoprotein (32). Dazu wird einer Patientin eine Blutprobe abgenommen, aus der das Serum separiert wird. Das Serum wird in eine Reaktionsmulde einer Mikrotiterplatte gefüllt. Die elektrische Nettoladung immunogener Epitope des Tandemrepeats von MUC1-Glykoprotein (32) wird durch drei zusätzliche Lysinreste, die bei neutralem oder leicht basischem pH-Wert positiv geladen sind, derart verändert, daß die veränderten MUC1-Epitope (34) bei neutralem oder leicht basischem pH-Wert einfach positiv geladen sind. Ferner wird für einen optischen Nachweis ein Farbstoff an die veränderten MUC1-Epitope gekoppelt. Zuckerspezifische, monoklonale anti-MUC1 Antikörper (58) werden mit den veränderten MUC1-Epitopen beladen und dem Serum zugegeben. Die beladenen Antikörper sind negativ geladen. Im Serum verdrängen die Tumormarker teilweise die veränderten MUC1-Epitope aus den Antigen-Bindungsstellen der monoklonalen Anti-MUC1 Antikörper. Der Antigen-Antikörper-Komplex aus MUC1-Tumormarker und monoklonalem anti-MUC1 Antikörper ist ebenfalls negativ geladen, während die freigesetzten veränderten MUC1-Epitope positiv geladen sind. Für den Nachweis wird ein elektrisches Feld zwischen einer Anode und einer Kathode durch das Serum geführt. Die farbstoffmarkierten und freigesetzten veränderten MUC1-Epitope sammeln sich an der Kathode, wo sie optisch nachgewiesen werden können. Ihre Konzentration ist ein Maß für die Konzentration der Tumormarker und damit für die Menge an Tumorzellen.

## Beschreibung

Die Erfindung betrifft ein Verfahren zum Nachweis eines ersten Moleküls in einer Lösung, wobei das erste Molekül in der Lösung an ein zweites Molekül binden kann. Ferner betrifft die Erfindung ein Verfahren zum Nachweis eines dritten Moleküls in einer Lösung, wobei das dritte Molekül in der Lösung an ein erstes Molekül und an ein zweites Molekül binden kann. Weiterhin betrifft die Erfindung eine Vorrichtung zum Nachweis eines Moleküls in einer Lösung. Außerdem betrifft die Erfindung ein farbstoffmarkiertes Peptid. Schließlich betrifft die Erfindung ein Verfahren zur Herstellung eines farbstoffmarkierten Peptids und dessen Verwendung.

Die Erfindung befaßt sich insbesondere mit dem Gebiet der Brustkrebsfrüherkennung und -verlaufskontrolle. Bei einem hohen Prozentsatz der Patientinnen mit Mammakarzinomen finden sich zum Zeitpunkt der Primäroperation und nach üblichen Therapien mikrometastatische Tumorzellen im Knochenmark. Die Heilungschancen dieser Patientinnen sind schlecht. Der möglichst frühe und sichere Nachweis der primären Tumorzellen bzw. der nach einer Behandlung verbliebenen (residuellen) bzw. mikrometastatischen Tumorzellen ist von herausragender Bedeutung für die Therapieplanung, die Verlaufskontrolle und die Heilungschancen einer jeden Tumorerkrankung.

Die Tumorzellen des Mammakarzinoms enthalten ein transmembranes Glykoprotein MUC1, welches im Vergleich zu gesunden Zellen unterglykosiliert ist (E. Petrakou, A. Murray, M.R. Price: "Epitope Mapping of Anti-MUC1 Mucin Protein Core Monocional Antibodies", Tumor Biology Band 19 (1998) S. 2 1-29; S. Kaul, S. Windecker, G. Bastert: "Monoclonal antibodies reactive with tumorassociated epitopes of breast mucin glycoproteins", Proceedings of the American Association of Cancer Research Band 30 (1989) S. 349-355). Durch die Unterglykosilierung wird MUC1 tumorspezifisch und wirkt in erhöhtem Maße immunogen. Ferner wird MUC1 überexprimiert und von den Tumorzellen verstärkt in Körperflüssigkeiten wie Serum und Aszites ausgeschieden. Die verstärkte Sekretion ermöglicht die Detektion der Tumorzellen des Mammakarzinoms. MUC1 dient damit als Tumormarker des Mammakarzinoms.

Bekannte Nachweismethoden für MUC1, wie der CA 15-3-Test, haben nur eine Empfindlichkeit von ca. 10⁻⁹ Mol MUC1 pro Liter. Sie eignen sich nur zur Therapieverlaufskontrolle, nicht aber zum Nachweis residueller Tumorzellen.

Der Nachweis residueller Tumorzellen erfolgt bisher durch Entnahme von Knochenmarkszellen aus dem Rückenmark. Dies ist sehr aufwendig und für die Patienten äußerst störend. Ferner basiert der Nachweis auf einer kleinen, lokalisierten Stichprobe des Beckenkamms.

Auch natürlich vorkommende anti-MUC1 Antikörper sind ein gutes prognostisches Kriterium beim frühen Mammakarzinom.

Ähnlich liegen die Probleme beim Nachweis anderer Tumormarker bzw. allgemein beim Nachweis von Molekülen im menschlichen Körper.

Aufgabe der Erfindung ist es, ein einfaches und hochempfindliches Verfahren, eine entsprechende Vorrichtung und einen Stoff zum Nachweis von Molekülen zu schaffen, sowie ein Verfahren zur Herstellung des Stoffs und dessen Verwendung anzugeben.

Diese Aufgabe wird erfindungsgemäß durch ein Verfahren mit den Merkmalen des Anspruchs 1 gelöst.

Das nachzuweisende erste Molekül (etwa ein anti-MUC1 Antikörper) kann z.B. zweifach negativ geladen sein. Es wandert dann im ungebundenen Zustand im elektrischen Feld zur Anode. Die der Lösung im Überschuß zugegebenen farbstoffmarkierten zweiten Moleküle (z.B. farbstoffmarkierte MUC1-Epitope) wurden derart modifiziert bzw. gewählt, daß ihre elektrische Nettoladung in der Lösung ein entgegengesetztes Vorzeichen hat, d.h. sie sind in diesem Beispiel positiv geladen. Dem Betrag nach ist ihre Ladung kleiner als die Ladung des ersten Moleküls, d.h. in diesem Beispiel etwa einfach positiv. Die zweiten Moleküle wandern daher im ungebundenen Zustand im elektrischen Feld entgegen den Antikörpern zur Kathode. Die farbstoffmarkierten zweiten Moleküle binden jedoch an die nachzuweisenden anti-MUC1 Antikörper, d.h. an die ersten Moleküle. Dabei kompensieren die negativen Landungen am Antikörper die positiven Ladungen des Epitops und das Konjugat, bestehend aus Epitop und Antikörper, wandert aufgrund der überschüssigen Negativladung zur Anode. D.h. gebundene und ungebundene farbstoffmarkierte MUC1-Epitope werden durch das elektrische Feld voneinander getrennt. Nur die gebundenen wandern zur Anode und können dort in sehr geringen Mengen neben einem 1000fachen Überschuß an freien, farbstoffmarkierten Epitopen nachgewiesen werden. Ihre Menge ist ein Maß für die Menge an anti-MUC1 Antikörpern in der Probe und damit ein Maß für die Anzahl von Mammakarzinom-Tumorzellen. Allgemein lassen sich somit die ersten Moleküle durch einen Nachweis des an die zweiten Moleküle gekoppelten Farbstoffs empfindlich nachweisen.

Durch die Trennung von freien und gebundenen zweiten Molekülen (z.B. MUC1-Epitopen) wird ein untergrundfreier und schneller Test geschaffen. Im Gegensatz zu den bekannten ELISA-Tests erfolgt beim erfindungsgemäßen Verfahren die Detektion ohne zusätzliche Spülschritte. Konzentrationsbestimmungen für die anti-MUC1 Antikörper (ersten Moleküle) sind erfindungsgemäß in unverdünnten Blutseren möglich. Es bedarf keiner Abnahme von Rückenmarksproben mehr. Das erfindungsgemäße Verfahren ist sicher und hochempfindlich. Es eignet sich daher insbesondere zur Tumorfrüherkennung. Es ist ca. 1000fach empfindlicher als bekannte ELISA-Tests. Die Empfindlichkeit kann dabei bis zum Nachweis eines einzelnen gebundenen Komplexes aus farbstoffmarkiertem Epitop (erstem Molekül) und anti-MUC1 Antikörper (zweitem Molekül) gehen. Das erfindungsgemäße Verfahren erreicht somit die maximale, theoretisch mögliche Empfindlichkeit. Dadurch kann eine wesentlich bessere Überlebenschance der Patientinnen gewährleistet werden.

Ferner wird die Aufgabe erfindungsgemäß durch ein Verfahren mit den Merkmalen des Anspruchs 2 gelöst.

Befinden sich in der Probe bzw. der Lösung ein drittes Molekül (in diesem Beispiel etwa tumorspezifische MUC1-Proteine), so werden die zweiten Moleküle (farbstoffmarkierten MUC1-Epitope) teilweise durch die MUC1-Proteine mit ihren Epitop-Regionen von den ersten Molekülen (anti-MUC1 Antikörpern) verdrängt. Der Antigen-Antikörper-Komplex aus MUC1-Proteine und anti-MUC1 Antikörper (Komplex aus erstem und drittem Molekül) ist negativ geladen. Er wandert im elektrischen Feld zur Anode. Aufgrund der in diesem Beispiel positiven Ladung der freigesetzten farbstoffmarkierten MUC1-Epitope (ersten Moleküle) wandern diese zur Kathode, wo sie nachgewiesen werden können. Ihre Menge ist ein Maß für die Menge an MUC1-Proteinen (dritten Molekülen) in der Probe und damit ein Maß für die Anzahl von Mammakarzinom-Tumorzellen in diesem Beispiel.

Erfindungsgemäß wurde somit ein kompetitiver, homogener Test geschaffen, der dieselben Vorteile hat, wie das Nachweisverfahren gemäß Anspruch 1.

Aufgrund der hohen Photostabilität wurden derartige Nachweise bisher mit Rhodaminfarbstoffen durchgeführt, die im Wellenlängenbereich von 480-580 nm absorbieren. In diesem Spektralbereich weisen biologische Proben, wie Seren, jedoch eine starke Eigenlumineszenz auf. Eine elegante Möglichkeit zur Reduzierung dieses Hintergrunds ist die Anregung mit rotem oder nahinfrarotem Licht. Die Eigenlumineszenz nimmt im Spektralbereich oberhalb 600 nm stark ab, da es nur wenige natürlich vorkommende Chromophore gibt, die in diesem Spektralbereich absorbieren. Der Einsatz von kostengünstigen Diodenlasern in der höchstempfindlichen Spektroskopie scheiterte jedoch lange am Mangel an geeigneten Farbstoffen, d.h. an Farbstoffen mit langweilig verschobener Absorption. In letzter Zeit wurden neue langweilig absorbierende Rhodamin- und Oxazinderivate entwickelt, die sich effizient mit Diodenlasers im Wellenlängenbereich von 620-670 nm anregen lassen. Ein Beispiel für diese Farbstoffe ist Cy5 (M. Sauer, K.-T. Han, R. Müller, A. Schulz, R. Tadday, S. Seeger, J. Wolfrum, J. Arden-Jacob, G. Deltau, N.J. Marx, K.H. Drexhage: "New fluorescent dyes for time-resolved biodiagnostics", Journal of Fluorescence Band 3 (1993) S. 131-139; M. Sauer, J. Arden-Jacob, G. Deltau, K.-T. Han, N.J. Marx, R. Müller, S. Nord, A. Schulz, S. Seeger, J. Wolfrum, C. Zander, K.H. Drexhage: "New dyes for the red spcetral region", Journal of Fluorescence Band 5 (1995) S. 247-261). Diese Farbstoffe verfügen über hohe Extinktionskoeffizienten und Fluoreszenzquantenausbeuten, so daß sie ideal für den hochempfindlichen Fluoreszenznachweis in biologischen Medien geeignet sind. Sie erlauben die Detektion und Identifikation einzelner farbstoffmarkierter Antikörper in unverdünnten Blutserumproben.

Eine weitere Verbesserung der Diskriminierung zwischen Fluoreszenzlicht und Hintergrundsignal kann erreicht werden, wenn die Farbstoffe durch zeitaufgelöste Fluoreszenzdetektion nachgewiesen werden, da die Farbstoffe charakteristische Fluoreszenzlebensdauern aufweisen, die sich von den Lumineszenzabklingzeiten des Hintergrunds unterscheiden. Mit zeitaufgelöster Fluoreszenzdetektion kann auch die Bindung von farbstoffmarkiertem Antikörper an den Tumormarker MUC1 durch das Auftreten einer verkürzten Fluoreszenzlebensdauer nach der Bindung online beobachtet werden.

Die Aufgabe wird außerdem erfindungsgemäß durch eine Vorrichtung mit den Merkmalen des Anspruchs 7 gelöst.

Für eine solche Vorrichtung können z.B. kommerziell erhältliche Mikrotiterplatten mit 96 Reaktionsmulden verwendet werden. Dies erlaubt eine hochgradige Parallelisierung und Automatisierung der durchzuführenden Tests. Die Elektroden erlauben es, die Wanderungsgeschwindigkeit und -richtung der Moleküle durch Anlegen einer geeigneten Spannung zu steuern.

In einer besonders eleganten Weiterbildung der Erfindung ist am oberen Rand jeder Reaktionsmulde eine Ringelektrode und im Inneren jeder Reaktionsmulde eine Elektrodenspitze angeordnet. Der Detektionsfokus wird dann knapp oberhalb der Elektrodenspitze gelegt, so daß die gesuchten Moleküle, die die innere Elektrode erreichen, effizient detektiert werden.

Die Aufgabe wird außerdem erfindungsgemäß durch ein farbstoffmarkiertes Peptid mit den Merkmalen des Anspruchs 9 gelöst.

Dieses Peptid besteht im Kern aus mindestens 3 aufeinanderfolgenden Aminosäuren der einem immunogenen Epitop (z.B. MUC1-Epitop) entsprechenden Peptidsequenz. Durch zusätzliche, bei neutralem oder leicht basischem pH-Wert positiv geladene Aminosäuren wird die Ladung des Epitops derart verändert, daß das veränderte Peptid bei neutralem oder leicht basischem pH-Wert mit entgegengesetztem Vorzeichen und dem Betrag nach weniger als die elektrische Nettoladung in der Lösung des Antikörpers geladen ist, im geschilderten Beispiel des humanen anti-MUC1 Antikörpers also weniger als zweifach positiv geladen ist. Alternativ kann es durch Ankoppeln eines geeignet geladenen Farbstoffs in seiner Ladung beeinflußt werden. Dadurch erhält es im elektrischen Feld die zur Durchführung der Verfahren nötige Bewegungsrichtung. Für den optischen Nachweis ist das in seiner Ladung veränderte Peptid ferner mit einem Farbstoff gekoppelt. Das farbstoffmarkierte Peptid ist ein wesentliches Hilfsmittel zur Durchführung der erfindungsgemäßen Verfahren.

Die Aufgabe wird schließlich erfindungsgemäß durch ein Verfahren zur Herstellung eines farbstoffmarkierten Peptids mit den Merkmalen des Anspruchs 13 sowie durch dessen Verwendung gemäß Anspruch 17 gelöst.

Weitere vorteilhafte Weiterbildungen der Erfindung sind in den Unteransprüchen gekennzeichnet.

Im folgenden wird die Erfindung anhand von Ausführungsbeispielen näher erläutert, die in den Figuren schematisch dargestellt sind. Gleiche Bezugsziffern in den einzelnen Figuren bezeichnen dabei gleiche Elemente. Im einzelnen zeigt:
- Fig. 1: eine schematische Darstellung der erfindungsgemäßen Vorrichtung;
- Fig. 2: eine schematische Darstellung der am Nachweis von anti-MUC1 Antikörpern beteiligten Moleküle, ihrer Ladungen und Wanderungsbewegung;
- Fig. 3: eine schematische Darstellung der am Nachweis von MUC1-Glykoprotein (Tumormarker) beteiligten Moleküle, ihrer Ladungen und Wanderungsbewegung; und
- Fig. 4: eine schematische Darstellung des farbstoffmarkierten Peptids gemäß dem bevorzugten Ausführungsbeispiel mit der zugehörigen Peptidsequenz.

### 1. Ausführungsbeispiel: Nachweis von anti-MUC1 Antikörpern

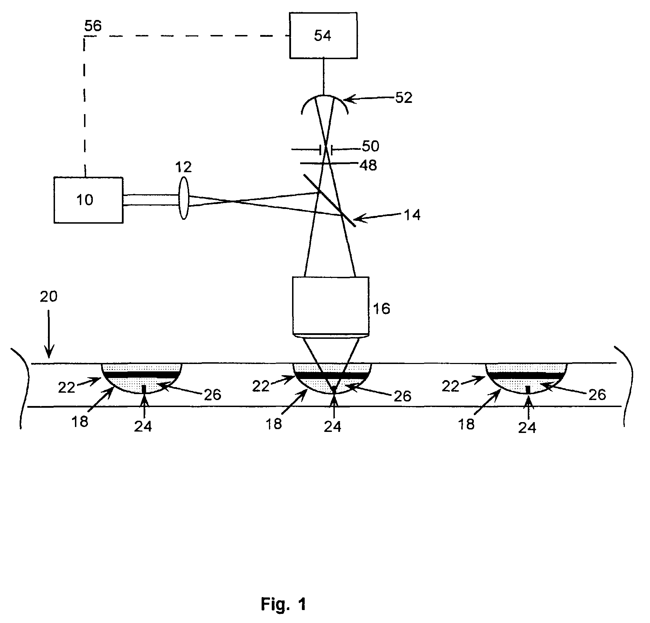

Fig. 1 zeigt einen gepulsten Halbleiterlaser 10 mit einer Wellenlänge von 635 nm. Sein Strahl wird wie bei einem üblichen konfokalen Aufbau über eine Linse 12 und einen Strahlteiler 14 in ein Mikroskopobjektiv 16 in Auflichtanordnung eingekoppelt. Das Mikroskopobjektiv 16 fokussiert den Laserstrahl in einer Reaktionsmulde 18 einer Mikrotiterplatte 20. In der Reaktionsmulde 18 befindet sich am oberen Rand eine
Ringelektrode 22 und am Fuß der Reaktionsmulde 18 eine Elektrodenspitze 24. Mit Hilfe des Mikroskopobjektivs 16 wird der Strahl eines Halbleiterlasers 10 so fokussiert, daß dessen Brennpunkt im Inneren der Lösung 18 nahe der Elektrodenspitze 24 liegt.

Einer Patientin wird zum Nachweis von eventuellen Tumorzellen des Mammakarzinoms Blut entnommen. Aus dem Blut wird Serum 26 abgetrennt. Das Serum 26 wird in eine Reaktionsmulde 18 der Mikrotiterplatte 20 eingefüllt. In der Reaktionsmulde 18 steht das Serum 26 in Kontakt mit der Ringelektrode 22 und der Elektrodenspitze 24 am Fuß der Reaktionsmulde 18.

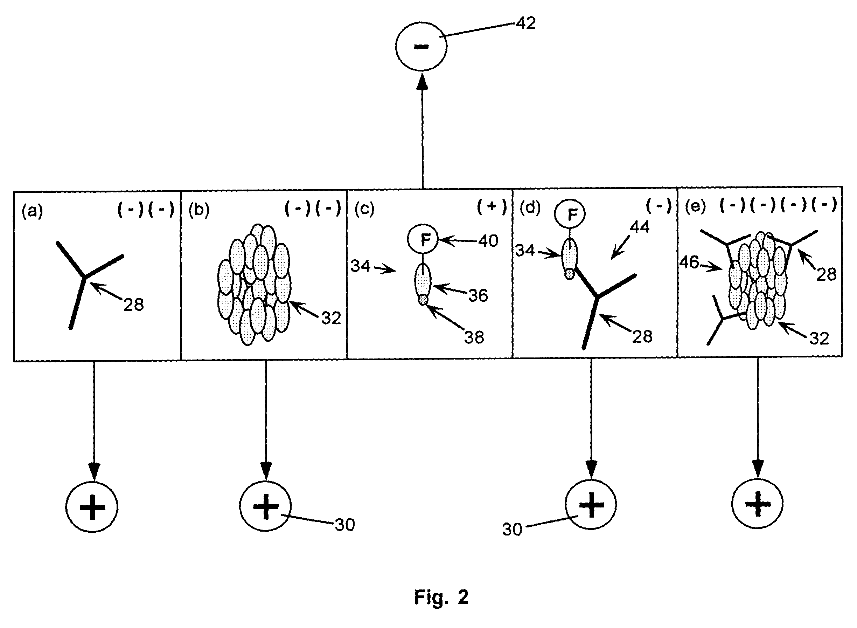

Im Folgenden wird auf Fig. 2 Bezug genommen. Fig. 2(a) zeigt einen humanen anti-MUC1 Antikörper 28, wie er sich beispielsweise im Blutserum 26 einer Patientin in der Reaktionsmulde 18 befinden kann. Seine Ladungen sind oben rechts in der Figur angedeutet. Der humane Antikörper 28 ist bei pH-Werten zwischen 7 und 9 z.B. zweifach negativ geladen und bewegt sich daher bei anliegender Spannung zur Anode 30.

Fig. 2(b) zeigt ein MUC1-Glykoprotein (Tumormarker) 32, das sich ebenfalls in dem Blutserum 26 der Patientin befinden kann. Seine Ladungen sind oben rechts in der Figur angedeutet. Der Tumormarker 32 ist bei neutralem oder leicht basischem pH-Wert ebenfalls zweifach negativ geladen und bewegt sich daher bei anliegender Spannung ebenfalls zur Anode 30.

Das MUC1-Glykoprotein ist durch 30 bis 90 tandemförmig hintereinander angeordnete Peptidsequenzen von je 20 Aminosäuren charakterisiert. Die 20 Aminosäuren umfassende Sequenz des Tandemrepeats lautet: PDTRPAPGS**TAPPAHGV**TSA, d.h. Prolin-Asparaginsäure-Threonin-Arginin-Prolin-Alanin-Prolin-Glycin-**Serin-** **Threonin-Atanin-Prolin-Prolin-Alanin-Histidin-Glycin-Valin-**Threonin-Serin-Alanin. Der fett gedruckte Abschnitt bildet das Epitop STAPPAHGV, d.h. Serin-Threonin-Alanin-Prolin-Prolin-Alanin-Histidin-Glycin-Valin. Das Epitop ist der für das Immunsystem erkennbare Schlüssel von MUC1, an das die meisten bisher entwickelten MUC1-spezifischen monoklonalen Antikörper binden.

Zum Nachweis der humanen anti-MUC1 Antikörper 28 muß dem Serum 26 noch die in Fig. 2(c) dargestellte Substanz 34 im Überschuß zugegeben werden. Fig. 2(c) zeigt ein das immunogene Epitop des Tandemrepeats von MUC1 enthaltende Peptid 36. Das Peptid 36 besteht aus einer variablen Anzahl von 3 bis 40 Aminosäuren. Im bevorzugten Ausführungsbeispiel wurden 20 Aminosäuren gewählt, und zwar genau die 20 Aminosäuren umfassende Sequenz des Tandemrepeats des MUC1-Glykoproteins. Die Sequenz des Peptids 36 lautet daher:
PDTRPAPGSTAPPAHGVTSA, d.h. Prolin-Asparaginsäure-Threonin-Arginin-Prolin-Alanin-Prolin-Glycin-Serin-Threonin-Alanin-Prolin-Prolin-Alanin-Histidin-Glycin-Valin-Threonin-Serin-Alanin.

Das Peptid 36 wird bei dem bevorzugten Ausführungsbeispiel durch drei zusätzliche Lysinreste 38 zweifach positiv aufgeladen. Es kann auch durch zusätzliche Argininreste bzw. Histidinreste positiv geladen werden. Allgemein eignen sich dazu Aminosäuren mit einem pK-Wert der Seitenkette von mehr als 9.

Die Synthese des veränderten Peptids 36 erfolgt mittels allgemein bekannter Verfahren.

Außerdem wird bei dem bevorzugten Ausführungsbeispiel an das N-terminale Ende des veränderten MUC1-Epitops 36, 38 ein Cy5-Molekül 40 angekoppelt. Dazu wird der Carbonsäurerest des Cy5 in einen Aktivester überführt und unter basischen Bedingungen unter Ausbildung einer Peptidbindung mit Aminofunktionen des Peptids verknüpft. Das Ankoppeln des Farbstoffs kann durch Veränderung der Reaktionsbedingungen auch über die Aminofunktionen der zusätzlichen Lysin- oder Argininreste bzw. über die Aminofunktionen zusätzlich angekoppelter Cysteinreste (-SH) erfolgen.

Cy5 ist bei pH-Werten zwischen 7 und 11 in der Lösung und nach der Kopplung elektrisch einfach negativ geladen. Die elektrische Nettoladung des farbstoffmarkierten MUC1-Epitops 34 ist daher einfach positiv. Bei Wahl eines Farbstoffs mit einer anderen elektrischen Nettoladung in der Lösung muß die Anzahl der zusätzlichen Aminosäuren und deren Ladung entsprechend gewählt werden, um die gewünschte elektrische Nettoladung in der Lösung des farbstoffmarkierten MUC1-Epitops 34 zu erhalten. Ggf. kann das Epitop durch Asparaginsäurereste oder Glutaminsäurereste negativ aufgeladen werden.

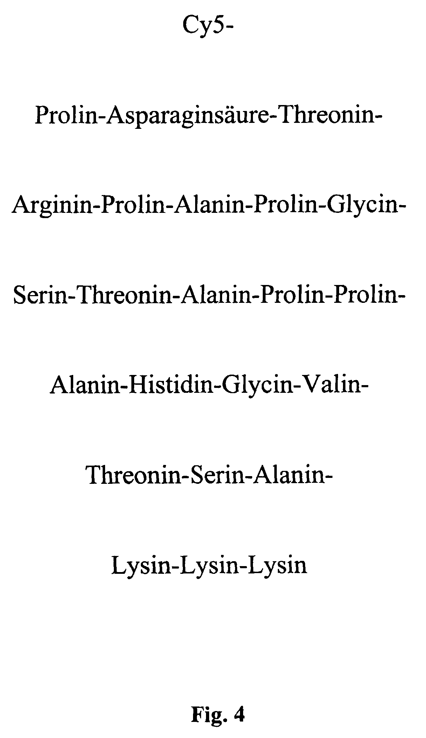

Das im bevorzugten Ausführungsbeispiel präparierte Molekül 34 ist in Fig. 4 im Detail gezeigt.

Die Ladung des im bevorzugten Ausführungsbeispiel präparierten Moleküls 34 ist oben rechts in Fig. 2(c) angedeutet. Bei pH-Werten zwischen 7 und 11 ist das Molekül 34 z.B. einfach positiv geladen. Es wandert daher zur Kathode 42.

Dies sind die einzelnen, getrennten Komponenten der Lösung. In der Lösung können sich jedoch zwei Komplexe bilden.

Fig. 2(d) zeigt den Antigen-Antikörper-Komplex 44 aus farbstoffmarkiertem MUC1-Epitop 34 und humanem anti-MUC1 Antikörper 28. Seine Ladung ist oben rechts in der Figur angedeutet. Der Komplex 44 ist bei pH-Werten zwischen 7 und 9 einfach negativ geladen und bewegt sich daher bei anliegender Spannung zur Anode 30.

Fig. 2(e) zeigt den Antigen-Antikörper-Komplex 46 aus MUC1-Tumormarker 32 und humanem anti-MUC1 Antikörper 28. Seine Ladung ist oben rechts in der Figur angedeutet. Der Komplex 46 ist bei pH-Werten zwischen 7 und 9 vierfach negativ geladen und bewegt sich daher bei anliegender Spannung zur Anode 30.

Das freie farbstoffmarkierte MUC1-Epitop 34 wird zur Kathode 42 gezogen, während alle anderen Substanzen, insbesondere auch das gebundene farbstoffmarkierte MUC1-Epitop 34, zur Anode 30 gezogen werden.

Im Folgenden wird wieder auf Fig. 1 Bezug genommen. Für den Nachweis humaner anti-MUC1 Antikörper stellen die Ringelektroden 22 am Rand der Reaktionsmulden 18 der Mikrotiterplatte 20 den negativen Pol (Kathode 42) dar. Dadurch wird gewährleistet, daß freie, farbstoffmarkierte MUC1-Epitope 34 nicht das Detektionsvolumen nahe der als Anode 30 arbeitenden Elektrodenspitze 24 erreichen. Nur die gebundenen farbstoffmarkierten MUC1-Epitope 34 erreichen aufgrund der überschüssigen Negativladung der humanen anti-MUC1 Antikörper 28 die Elektrodenspitze 24 nahe dem Detektionsvolumen.

Die Detektion des Fluoreszenzsignals erfolgt konfokal über das oben genannte Mikroskopobjektiv 16. Das vom Mikroskopobjektiv 16 gesammelte Licht passiert den Strahlteiler 14. Dieser ist derart ausgebildet, daß er Licht bei der Anregungswellenlänge von 635 nm reflektiert und das rotverschobene Fluoreszenzlicht transmittiert. Zur weiteren spektralen Diskriminierung passiert das Fluoreszenzlicht noch einen dielektrischen Bandpaßfilter 48. Zur räumlichen Diskriminierung von Hintergrundlicht, das außerhalb des Laserfokus entsteht, wird eine Lochblende 50 verwendet. Das gesammelte Fluoreszenzlicht wird mittels einer Avalanche Photodiode 52 detektiert und in elektrische Impulse umgesetzt.

Die Avalanche Photodiode 52 ist geeignet, einzelne Photonen mit eine zeitlichen Auflösung unterhalb von 1 ns nachzuweisen. Sie ist daher für zeitaufgelöstes Einzelphotonenzählen geeignet.

Die Signale der Avalanche Photodiode 52 werden in einer Datenverarbeitungseinheit 54 weiterverarbeitet, die über eine Verbindungsleitung 56 mit dem Halbleiterlaser 10 verbunden ist, um von diesem Triggerimpulse zu erhalten.

Der Laserfokus in der Reaktionsmulde 18 knapp oberhalb der Elektrodenspitze 24 hat ein Volumen im Femtoliterbereich. Das geringe Detektionsvolumen erlaubt die Detektion individueller farbstoffmarkierter Komplexe. Die Konzentration von MUC1-Komplexen in der Lösung kann daher um mehrere Größenordnungen unter 10⁻⁹ Mol liegen, z.B. bei 10⁻¹³ Mol.

### 2. Ausführungsbeispiel: Nachweis von MUC1-Glykoprotein (Tumormarker)

Der Nachweis von MUC1-Glykoprotein (Tumormarker) 32 erfolgt in vielen Schritten analog zum Nachweis von humanen anti-MUC1 Antikörpern 28 gemäß dem ersten Ausführungsbeispiel. Es werden daher nur die Unterschiede herausgestellt.

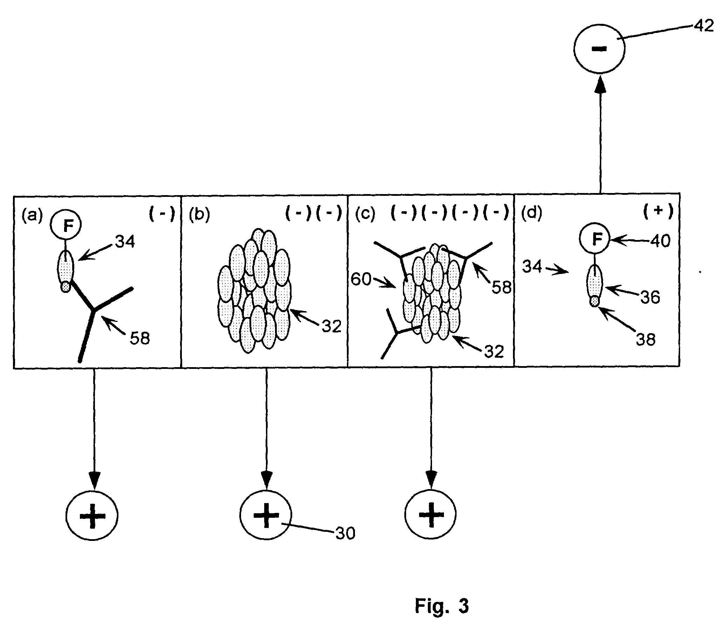

Im Folgenden wird auf Fig. 3 Bezug genommen. Fig. 3(a) zeigt einen zuckerspezifischen, monoklonalen anti-MUC1 Antikörper 58, z.B. BM7 oder BM8 (E. Petrakou, A. Murray, M.R. Price: "Epitope Mapping of Anti-MUC1 Mucin Protein Core Monoclonal Antibodies", Tumor Biology Band 19 (1998) S. 21-29), der mit farbstoffmarkiertem MUC1-Epitop 34 beladen ist. Dieser Komplex wird dem Serum in der Reaktionsmulde 18 zugegeben. Seine Ladungen sind oben rechts in der Figur angedeutet. Der farbstoffbeladene Antikörper 58 ist bei pH-Werten zwischen 7 und 9 einfach negativ geladen und bewegt sich daher bei anliegender Spannung zur Anode 30.

Im Serum befindet sich bei Patientinnen mit Tumorzellen ferner das in Fig. 3(b) dargestellte MUC1-Glykoprotein (Tumormarker) 32. Fig. 3(b) entspricht Fig. 2(b).

Im Serum verdrängen nun die Tumormarker 32 - in Abhängigkeit von ihrer Konzentration - die farbstoffmarkierten MUC1 -Epitop 34 teilweise aus den Antigen-Bindungsstellen der monoklonalen anti-MUC1 Antikörper 58. Sie bilden dabei den in Fig. 3(c) dargestellten Antigen-Antikörper-Komplex 60 aus MUC1-Tumormarker 32 und monoklonalem anti-MUC1 Antikörper 58. Fig. 3(c) entspricht im übrigen Fig. 2(e).

Durch die kompetitive Verdrängungsreaktion werden farbstoffmarkierte MUC1-Epitop 34 freigesetzt, wie sie in Fig. 3(d) dargestellt sind. Fig. 3(d) entspricht Fig. 2(c). Die freigesetzten farbstoffmarkierten MUC1-Epitop 34 sind bei neutralem oder leicht basischem pH-Wert positiv geladen und wandern zur Kathode 42. Alle anderen dargestellten Substanzen wandern zur Anode 30.

Die Ringelektrode 22 in den Reaktionsmulden 18 der Mikrotiterplatte 20 bildet beim zweiten Ausführungsbeispiel daher den positiven Pol (Anode 30), während die Elektrodenspitze 24 am Fuß der Reaktionsmulden 18 die Kathode 42 bildet. Gegenüber dem ersten Ausführungsbeispiel liegt somit eine Umpolung vor. Der Antigen-Antikörper-Komplex 60 und der farbstoffbeladene Antikörper 58 wandern aufgrund ihrer negativen Überschußladung zur Ringelektrode 22. Nur die freigesetzten farbstoffmarkierten MUC1-Epitop 34 wandern aufgrund ihrer positiven Landung zur Spitzenelektrode 24 nahe dem Detektionsvolumen.

Dort können sie optisch nachgewiesen werden. Ihre Konzentration ist ein Maß für die Konzentration an Tumormarkern 32. Diese wiederum sind ein Maß für die Menge an Tumorzellen im Körper der Patientin.

Im Rahmen der Erfindung sind zahlreiche Abwandlungen und Weiterbildungen der beschriebenen Ausführungsbeispiele verwirklichbar. So können die erfindungsgemäßen Verfahren außer in Mikrotiterplatten auch in Mikrokapillaren mit einem Innendurchmesser an der Spitze von ca. 0,5 µm durchgeführt werden, wie sie in der DE 197 42 227 A1 beschrieben sind. Derartige Mikrokapillaren erlauben die sichere Detektion eines jeden farbstoffmarkierten Moleküls oder Komplexes.

Auch ist die Bindung zwischen dem ersten, zweiten und dritten Molekül nicht auf die Bindung zwischen einem Antikörper und dem zugehörigen Antigen bzw. Epitop beschränkt. Ebenso kann die Bindung durch eine Biotin-Streptavidin-Bindung realisiert werden. Jede andere Bindung, die prinzipiell für einen kompetitiven Test geeignet ist, kann verwendet werden. Daher müssen die ersten, zweiten und dritten Moleküle auch keine Proteine sein.

Die in Fig. 1 dargestellte Vorrichtung kann außer mit den Ringelektroden 22 auch mit zwei spitzen Elektroden realisiert werden, einer Anode und einer Kathode, die von unten durch die Mikrotiterplatte in die Reaktionsmulde 18 gesteckt werden. Dies ist insbesondere bei Mikrotiterplatten aus Polycarbonat möglich.

In dem bevorzugten Ausführungsbeispiel war der humane anti-MUC1 Antikörper 28 zweifach negativ geladen. Andere Antikörper sind positiv geladen. Generell kann aber jeder Antikörper negativ aufgeladen werden, so daß er eine negative Überschußladung hat. Hierzu kann an die NH₂-Funktionen des Antikörpers ein Succinimidylester (etwa ein bifunktionelles wie GMBS) gekoppelt werden. Dadurch werden die NH₂-Funktionen in COO⁻/H⁺-Funktionen überführt.

### Bezugszeichenliste

- 10: Halbleiterlaser
- 12: Linse
- 14: Strahlteiler
- 16: Mikroskopobjektiv
- 18: Reaktionsmulde
- 20: Mikrotiterplatte
- 22: Ringelektrode
- 24: Elektrodenspitze
- 26: Serum
- 28: humaner anti-MUC1 Antikörper
- 30: Anode
- 32: MUC1-Glykoprotein (Tumormarker)
- 34: farbstoffmarkiertes MUC1-Epitop
- 36: Peptid, das das immunogene Epitop des Tandemrepeats von MUC1-Glykoprotein 32 enthält
- 38: Lysinreste
- 40: Farbstoff Cy5
- 42: Kathode
- 44: Antigen-Antikörper-Komplex 44 aus farbstoffmarkiertem MUC1-Epitop 36 und humanem anti-MUC1 Antikörper 28
- 46: Antigen-Antikörper-Komplex 46 aus MUC1-Tumormarker 32 und humanem anti-MUC1 Antikörper 28
- 48: Bandpaßfilter
- 50: Lochblende
- 52: Avalanche-Photodiode
- 54: Datenverarbeitungseinheit
- 56: Verbindungsleitung zwischen Datenverarbeitungseinheit 54 und Halbleiterlaser 10
- 58: zuckerspezifischer, monoklonaler anti-MUC1 Antikörper
- 60: Antigen-Antikörper-Komplex aus MUC1-Tumormarker 32 und monoklonalem anti-MUC1 Antikörper 58

Sequenz.txt
## Patentansprüche

1. Verfahren zum Nachweis eines ersten Moleküls (28) in einer Lösung (26), wobei das erste Molekül in der Lösung an ein zweites Molekül (36) binden kann, mit folgenden Schritten:
a) ein Farbstoff (40) wird an das zweite Molekül (36) gekoppelt;
b) die elektrische Nettoladung in der Lösung (26) des farbstoffinarkierten zweiten Moleküls (36) wird mit entgegengesetztem Vorzeichen und dem Betrag nach kleiner als die elektrische Nettoladung in der Lösung des ersten Moleküls (28) gewählt;
c) das farbstoffmarkierte zweite Molekül (34) wird der Lösung (26) zugegeben;
d) ein elektrisches Feld wird zwischen einer Anode (30) und einer Kathode (42) durch die Lösung (26) geführt; und
e) der Farbstoff (40) wird, falls die elektrische Nettoladung in der Lösung des ersten Moleküls (28) negativ ist, an der Anode (30) und, falls die elektrische Nettoladung in der Lösung des ersten Moleküls (28) positiv ist, an der Kathode (42) optisch nachgewiesen.

2. Verfahren zum Nachweis eines dritten Moleküls (32) in einer Lösung (26), wobei das dritte Molekül (32) in der Lösung an ein erstes Molekül (58) und an ein zweites Molekül (36) binden kann, mit folgenden Schritten:
f) ein Farbstoff (40) wird an das zweite Molekül (36) gekoppelt;
g) die elektrische Nettoladung in der Lösung (26) des farbstoffmarkierten zweiten Moleküls (36) wird mit entgegengesetztem Vorzeichen und dem Betrag nach kleiner als die elektrische Nettoladung in der Lösung des ersten Moleküls (58) gewählt;
h) gebundene Komplexe aus erstem Molekül (58) und zweitem Molekül (34) werden hergestellt;
i) die gebundenen Komplexe werden der Lösung (26) zugegeben;
j) ein elektrisches Feld wird zwischen einer Anode (30) und einer Kathode (42) durch die Lösung (26) geführt; und
k) der Farbstoff (40) wird, falls die elektrische Nettoladung in der Lösung des ersten Moleküls (58) negativ ist, an der Kathode (42) und, falls die elektrische Nettoladung des ersten Moleküls (28) positiv ist, an der Anode (30) optisch nachgewiesen.

3. Verfahren nach Anspruch 1 oder 2, **dadurch gekennzeichnet**,
daß als zweites Molekül (36) ein Peptid gewählt wird; und
daß in Schritt b) bzw. g) die elektrische Nettoladung in der Lösung (26) des Peptids (36) abhängig von der elektrischen Nettoladung in der Lösung des ersten Moleküls (28, 58) durch zusätzliche, bei neutralem oder leicht basischem pH-Wert positiv oder negativ geladene Aminosäuren (38) verändert wird.

4. Verfahren nach einem der Ansprüche 1 bis 3, **dadurch gekennzeichnet**, daß in Schritt b) bzw. g) die elektrische Nettoladung in der Lösung (26) des zweiten Moleküls (36) abhängig von der elektrischen Nettoladung in der Lösung des ersten Moleküls (28, 58) dadurch verändert wird, daß der Farbstoff (40) derart gewählt wird, daß seine elektrische Nettoladung in der Lösung positiv oder negativ ist.

5. Verfahren nach einem der Ansprüche 1 bis 4, **dadurch gekennzeichnet**,
daß als erstes Molekül (28, 58) ein Antikörper und als drittes Molekül (32) ein zugehöriges Antigen gewählt wird; und
daß das zweite Molekül (36) als Peptid gewählt wird, dessen Peptidsequenz mindestens 3 aufeinanderfolgende Aminosäuren der einem immunogenen Epitop entsprechenden Peptidsequenz des Antigens (32) aufweist.

6. Verfahren nach Anspruch 5, **dadurch gekennzeichnet**, daß die elektrische Nettoladung in der Lösung (26) des Peptids (36) durch 2 bis 4 zusätzliche Aminoreste (38) positiv aufgeladen wird.

7. Vorrichtung zum Nachweis eines Moleküls (28, 32) in einer Lösung (26) mit:
l) einer Lichtquelle (10) einer vorgegebenen Wellenlänge;
m) einer Mikrotiterplatte (20) mit einer Mehrzahl von Reaktionsmulden (18);
n) einem Paar von Elektroden (22, 24) in jeder Reaktionsmulde (18); und
o) einer auf ein Volumen nahe einer der beiden Elektroden (22, 24) gerichteten optischen Einrichtung (12, 14, 16, 48, 50, 52, 54, 56) zum Detektieren von Licht aus dem Volumen.

8. Vorrichtung nach Anspruch 7, **dadurch gekennzeichnet**,
daß am oberen Rand jeder Reaktionsmulde (18) eine Ringelektrode (22) und im Inneren jeder Reaktionsmulde eine Elektrodenspitze (24) angeordnet ist; und
daß die optische Einrichtung (12, 14, 16, 48, 50, 52, 54, 56) auf ein Volumen nahe der Elektrodenspitze (24) ausgerichtet ist.

9. Farbstoffmarkiertes Peptid (34) herstellbar durch folgende Schritte:
p) die einem immunogenen Epitop eines Antigens (32) entsprechende Peptidsequenz (36) wird bestimmt;
q) ein Peptid (36) wird aus mindestens 3 aufeinanderfolgenden Aminosäuren der dem immunogenen Epitop entsprechenden Peptidsequenz synthetisiert;
r) ein Farbstoff (40) wird an das Peptid (36) gekoppelt;
s) die elektrische Nettoladung eines in einer Lösung (26) gelösten Antikörpers (28, 58) gegen das Antigen (32) und die elektrische Nettoladung in der Lösung des farbstoffmarkierten Peptids (36) wird bestimmt; und
t) die elektrische Nettoladung in der Lösung (26) des farbstoffmarkierten Peptids (36) wird mit entgegengesetztem Vorzeichen und dem Betrag nach kleiner als die elektrische Nettoladung in der Lösung des Antikörpers (28, 58) gewählt.

10. Farbstoffmarkiertes Peptid (34) nach Anspruch 9, **dadurch gekennzeichnet**, daß in Schritt t) die elektrische Nettoladung in der Lösung (26) des Peptids (36) abhängig von der elektrischen Nettoladung in der Lösung des Antikörpers (28, 58) durch zusätzliche, bei neutralem oder leicht basischem pH-Wert positiv oder negativ geladene Aminosäuren (38) verändert wird.

11. Farbstoffmarkiertes Peptid (34) nach Anspruch 9 oder 10, **dadurch gekennzeichnet**, daß in Schritt t) die elektrische Nettoladung in der Lösung (26) des Peptids (36) abhängig von der elektrischen Nettoladung in der Lösung des Antikörpers (28, 58) dadurch verändert wird, daß der Farbstoff (40) derart gewählt wird, daß seine elektrische Nettoladung in der Lösung positiv oder negativ ist.

12. Farbstoffmarkiertes Peptid (34) nach einem der Ansprüche 9 bis 11, **dadurch gekennzeichnet**, daß die elektrische Nettoladung in der Lösung (26) des Peptids (36) durch 2 bis 4 zusätzliche Aminoreste (38) positiv aufgeladen ist.

13. Verfahren zur Herstellung eines farbstoffmarkierten Peptids (36) mit folgenden Schritten:
u) die einem immunogenen Epitop eines Antigens (32) entsprechende Peptidsequenz (36) wird bestimmt;
v) ein Peptid (36) wird aus mindestens 3 aufeinanderfolgenden Aminosäuren der dem immunogenen Epitop entsprechenden Peptidsequenz synthetisiert;
w) ein Farbstoff (40) wird an das Peptid (36) gekoppelt;
x) die elektrische Nettoladung eines in einer Lösung (26) gelösten Antikörpers (28, 58) gegen das Antigen (32) und die elektrische Nettoladung in der Lösung des farbstoffmarkierten Peptids (36) wird bestimmt; und
y) die elektrische Nettoladung in der Lösung (26) des farbstoffhiarkierten Peptids (36) wird mit entgegengesetztem Vorzeichen und dem Betrag nach kleiner als die elektrische Nettoladung in der Lösung des Antikörpers (28, 58) gewählt.

14. Verfahren nach Anspruch 13, **dadurch gekennzeichnet**, daß in Schritt y) die elektrische Nettoladung in der Lösung (26) des Peptids (36) abhängig von der elektrischen Nettoladung in der Lösung des Antikörpers (28, 58) durch zusätzliche, bei neutralem oder leicht basischem pH-Wert positiv oder negativ geladene Aminosäuren (38) verändert wird.

15. Verfahren nach Anspruch 13 oder 14, **dadurch gekennzeichnet**, daß in Schritt y) die elektrische Nettoladung in der Lösung (26) des Peptids (36) abhängig von der elektrischen Nettoladung in der Lösung des Antikörpers (28, 58) dadurch verändert wird, daß der Farbstoff (40) derart gewählt wird, daß seine elektrische Nettoladung in der Lösung positiv oder negativ ist.

16. Verfahren nach einem der Ansprüche 13 bis 15, **dadurch gekennzeichnet**, daß die elektrische Nettoladung in der Lösung (26) des Peptids (36) durch 2 bis 4 zusätzliche Aminoreste (38) positiv aufgeladen wird.

17. Verwendung des farbstoffmarkierten Peptids (34) nach einem der Ansprüche 9 bis 12 zum Nachweis eines ersten oder dritten Moleküls (28, 32) in einer Lösung (26) mittels des Verfahrens nach einem der Ansprüche 1 bis 6.


Sequenzprotokoll

<110> Sauer, Markus; Wolfrum, Jürgen

<120> Verfahren, Vorrichtung und farbstoffmarkiertes Peptid zum
Nachweis eines Moleküls, sowie Verfahren zur Herstellung eines
farbstoffmarkierten Peptids und dessen Verwendung

<140> DE 199 52 160.3
<141> 1999-10-29

<160> 3

<210> 1
<211> 9
<212> PRT
<213> Homo Sapiens

<300>
<301> Petrakou, E.; Murray, A.; Price, M.R.
<302> Epitope Mapping of Anti-MUC1 Mucin Protein Core Monoclonal
Antibodies
<303> Tumor Biology
<304> 19
<306> 21-29
<307> 1998

<300>
<301> Kaul, S.; Windecker, S.; Bastert, G.
<302> Monoclonal antibodies reactive with tumorassociated epitopes
of breast mucin glycoproteins
<303> Proceedings of the American Association of Cancer Research
<304> 30
<306> 349-355
<307> 1989

<400> 1

Ser Thr Ala Pro Pro Ala His Gly Val
1               5

<210> 2
<211> 20
<212> PRT
<213> Homo Sapiens

<300>
<301> Petrakou, E.; Murray, A.; Price, M.R.
<302> Epitope Mapping of Anti-MUC1 Mucin Protein Core Monoclonal
Antibodies

Sequenz.txt
<303> Tumor Biology
<304> 19
<306> 21-29
<307> 1998

<300>
<301> Kaul, S.; Windecker, S.; Bastert, G.
<302> Monoclonal antibodies reactive with tumorassociated epitopes
of breast mucin glycoproteins
<303> Proceedings of the American Association of Cancer Research
<304> 30
<306> 349-355
<307> 1989

<400> 2

Pro Asp Thr Arg Pro Ala Pro Gly Ser Thr Ala Pro Pro Ala His Gly
 1               5                   10
 15

Val Thr Ser Ala
                20


<210> 3

<211> 23
<212> PRT
<213> künstliche Sequenz

<220>
<221> VARIANT
<222> 21 - 23

<300>
<301> Petrakou, E.; Murray, A.; Price, M.R.
<302> Epitope Mapping of Anti-MUC1 Mucin Protein Core Monoclonal
Antibodies
<303> Tumor Biology
<304> 19
<306> 21-29
<307> 1998

<300>
<301> Kaul, S.; Windecker, S.; Bastert, G.
<302> Monoclonal antibodies reactive with tumorassociated epitopes
of breast mucin glycoproteins
<303> Proceedings of the American Association of Cancer Research
<304> 30
<306> 349-355
<307> 1989

Sequenz.txt
<400> 3

Pro Asp Thr Arg Pro Ala Pro Gly Ser Thr Ala Pro Pro Ala His Gly
1               5                   10
15

Val Thr Ser Ala Lys Lys Lys
            20